# NEW EUROPEAN PATENT SPECIFICATION

(11) **EP 1 938 777 B2**
(45) Date of publication and mention of the opposition decision: **02.12.2015**
(45) Mention of the grant of the patent: 31.10.2012
(21) Application number: 08101875.6
(22) Date of filing: 28.06.2001
(51) Int. Cl.: A61F 13/15, A61F 13/49

(54) **Method for manufacturing disposable diaper**
Herstellungsverfahren für Einweg-Gebrauchsartikel
Procédé pour la fabrication d'un article usagé jetable

(30) Priority: 04.08.2000 JP 2000237455
(43) Date of publication of application: 02.07.2008
(62) Divisional of application: 01114822.8
(73) Proprietor: Zuiko Corporation, Settsu-shi, Osaka 566-0045 (JP)
(72) Inventor: TACHIBANA, Ikuo, Hyogo Osaka 662-0072 (JP); INOUE, Kiyofumi, Hyogo Hyogo 663-8003 (JP); ICHIURA, Yuzo, Osaka Osaka 567-0845 (JP); TANAKA, Yoshinari, Osaka Osaka 564-0032 (JP); NAKAKADO, Masaki, Osaka Osaka 573-0065 (JP); TANAKA, Satoshi, Osaka Osaka 563-0043 (JP)
(74) Representative: Schwabe - Sandmair - Marx

(56) References cited:
- EP-A1- 1 188 427
- EP-A2- 1 179 330
- WO-A-00/76444
- WO-A1-99/32164
- WO-A1-99/42067
- JP-A- H10 131 014
- JP-A- 2000 026 015
- JP-A- 2000 026 015
- US-A- 5 622 581
- US-A- 5 660 657
- PATENT ABSTRACTS OF JAPAN vol. 016, no. 455 (C-0987), 22 September 1992 (1992-09-22) & JP 04 161152 A (KAO CORP), 4 June 1992 (1992-06-04)
- 'Longman Dictionary of the English Language', 1991, LONGMAN GROUP UK LIMITED, ESSEX page 511
- Translation of priority document JP 2000-237455

## Description

### BACKGROUND OF THE INVENTION

### FIELD OF THE INVENTION:

The present invention relates to a method for manufacturing a disposable worn article using an elastic sheet.

### DESCRIPTION OF THE RELATED ART:

Japanese Laid-Open Patent Publication No. 2000-26015 discloses a method for cutting off only an elastic member without damaging a base material sheet by using a roll cutter having a ridge-shaped blade thereon.

However, Laid-Open Patent Publication No. 2000-26015 fails to disclose sealing the base material sheet while cutting off the elastic member, improving the air permeability of the base material sheet while cutting off the elastic member, etc.

WO 00/76444 published after the priority date of the present application discloses a production method for disposable under-shorts comprising the steps of: transferring continuous materials of a pair of non-woven fabric sheets forming a fit gather laminate and a continuous material of an elastic member provided therebetween in a longitudinal direction thereof while intermittently securing the continuous material of the elastic member on the non-woven fabric sheets; cutting off the elastic member along with the non-woven fabric sheets along a non-secured area thereof; securing a reinforcement sheet on a lower surface side of the fit gather laminate at a position corresponding to the non-secured area of the elastic member while providing an absorbent on an upper surface side of the fit gather laminate; and providing a back sheet on a lower surface side of the reinforcement sheet.

### SUMMERY OF THE INVENTION

A method for manufacturing a combined web for a disposable worn article of the present invention includes: a first step of applying an adhesive on at least one of a first web and a second web; a second step of sandwiching an elastic member between the first and second webs and combining the first and second webs and the elastic member together, thereby producing a combined web ; wherein the adhesive is placed between the first web and the second web and a third step of cutting off a portion of at least one of the first and second webs, and the elastic member by passing the combined web between an embossing roll having a plurality of protrusions which are arranged in the axial direction of the embossing roll at intervals and a counter roll. The interval of the protrusions in a direction of a rotation axis of the embossing roll is about 1 mm to 25 mm.

In another embodiment of the invention, the third step is performed by passing the combined web between an embossing roll having a portion of lattice portions and a counter roll.

In still another embodiment of the invention: at least the first web includes a design area having at least one of a graphical design, a symbol and a character printed thereon; and the elastic member located on at least a portion of the design area is cut off in the third step.

In still another embodiment of the invention: at least the second web includes an area on which a member is to be adhered; and the elastic member located under at least a portion of the area is cut off in the third step.

In still another embodiment of the invention, the elastic member is at least one of a string rubber, a flat rubber and a meshed rubber.

### BRIEF DESCRIPTION OF THE DRAWINGS

FIG. **1** is a perspective view illustrating one embodiment of the method of the present invention.
Each of FIG. **2A,** FIG. **2B** and FIG. **2C** is a diagram illustrating an example of a disposable worn article using a combined web.
Each of FIG. **3A** and FIG. **3B** is a diagram illustrating an example of a staggered emboss pattern.
FIG. **4** is a diagram illustrating how elastic members are cut off.
FIG. **5** is a diagram illustrating how elastic members are cut off.
FIG. **6** is a diagram illustrating an example of a lattice emboss pattern.
FIG. **7A** is a diagram illustrating an example of an adhesive applicator, and each of FIG. **7B** and FIG. **7C** is a diagram illustrating an example of a conductive portion thereof.

### DESCRIPTION OF THE PREFERRED EMBODIMENTS

A first embodiment of the present invention will now be described with reference to the accompanying drawings.

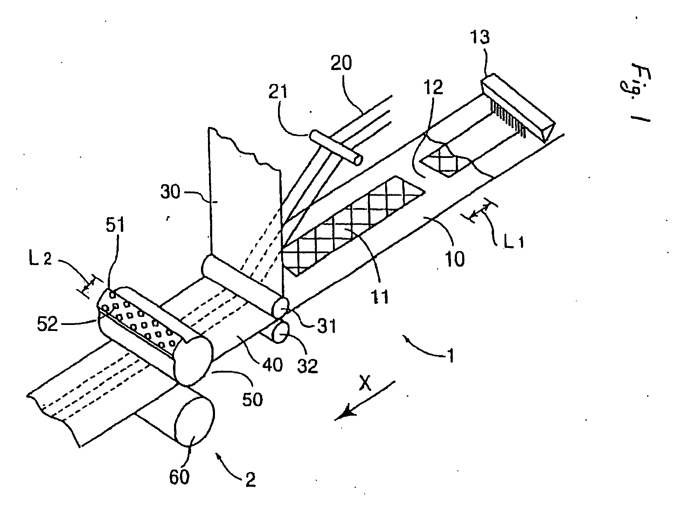

FIG. **1** is a diagram illustrating a manufacturing apparatus **1** for manufacturing a disposable worn article according to the first embodiment.

The manufacturing apparatus **1** includes an adhesive applicator **13** for applying an adhesive on a first sheet **10,** a guide **21** for guiding an elastic member **20** between the first sheet **10** and a second sheet **30,** press rolls **31** and **32** for pressing together the first sheet **10** and the second sheet **30** into a combined web **40,** and a processing section **2** for cutting off at least a part of, or reducing the shrinking force of, the elastic member **20** of the combined web **40** produced through the press rolls **31** and **32.** The adhesive may be applied on the second sheet **30.**

The adhesive applicator **13** is capable of applying an adhesive on the first sheet **10,** which has a strip shape of a predetermined width and which is advanced in the X direction (the direction of the production flow), so that an adhesive portion **11** and a non-adhesive portion **12** are provided on the first sheet **10.** In terms of the air permeability of the combined web **40,** it is preferred that the adhesive applicator **13** is a spray coater, a curtain coater, a spiral coater, or the like. The amount of adhesive is preferably about 1 g/m² to about 20 g/m². The adhesive may be a hot melt. A specific example of the adhesive applicator **13** will be described later.

The elastic member **20** under a predetermined tension is supplied to the guide **21.** The elastic member **20** may be in the form of a plurality of lines, as illustrated in FIG. **1****,** or a mesh (e.g., elastomeric netting such as REBOUND^{(R)} of CONWED PLASTICS may be used). The elastic member **20** may be a string rubber or a flat rubber. The guide **21** is capable of reciprocating transversely of the direction of the production flow. In such a case, the guide **21** is capable of guiding the elastic member **20** between the first sheet **10** and the second sheet **30** so that the elastic member **20** is drawn in a non-linear line. A predetermined tension is applied to the elastic member **20** supplied to the guide **21** by means of a tension roll (not shown).

The processing section **2** includes an embossing roll **50** and a counter roll **60** facing the embossing roll **50.** The combined web **40** is inserted between the embossing roll **50** and the counter roll **60,** and the processing section **2** cuts off at least a part of, or reduces, the shrinking force of, the elastic member **20.** The embossing roll **50** includes an embossing section **51** having a plurality of protrusions. The protrusions may generate heat as will be described later. In such a case, the amount of heat to be generated is determined by the distance between the embossing roll **50** and the counter roll **60,** the shape and/or the size of each protrusion, the material, the cross-sectional area and/or the shape of the elastic member **20,** and/or the speed at which the combined web **40** is advanced.

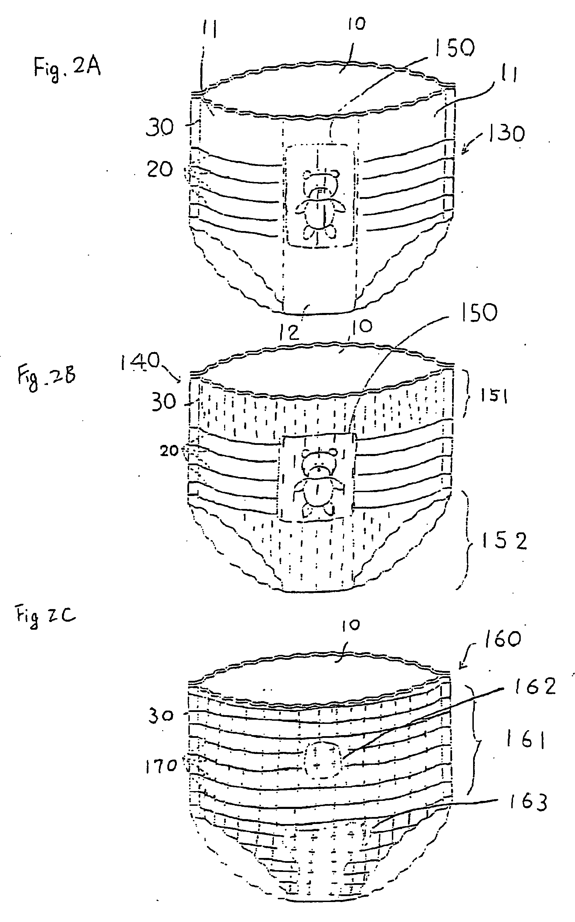

FIG. **2A** to FIG. **2C** illustrate an exemplary disposable worn article made of the combined web **40,** which has been passed through the processing section **2.** The disposable worn article illustrated in FIG. **2A** to FIG. **2C** (e.g., disposable underpants or a disposable diaper; hereinafter referred to simply as "underpants") includes a design area **150** having a graphical design or characters printed thereon. If the shrinking force of a part of the elastic member **20** on the design area **150** is not reduced or eliminated, the design area **150** will be wrinkled by the force, thereby deteriorating the appearance of the article.

Also in cases where a label having a graphical design or characters printed thereon is attached to the combined web **40,** the label will have wrinkles due to the shrinkage of the elastic member **20,** thereby deteriorating the appearance of the article. Besides, such wrinkles make it difficult to adhere, on the combined web **40,** a member such as a label, a tape used for fixing a diaper, a tape used when disposing of the article having the combined web **40,** etc. Even if such a member is successfully adhered on the combined web **40** while the wrinkles are being smoothed out, it is difficult to keep the adhesion between the combined web **40** and the member for a long period of time due to the shrinkage of the elastic member **20.**

Underpants **130** include the adhesive portion **11**, the non-adhesive portion **12** which has substantially the same width as that of the design area **150**, and the part of the elastic member **20** on the design area **150** is cut off. The shrinkage of the cut elastic member **20** stops in the vicinity of the boundary between the adhesive portion **11** and the non-adhesive portion **12**. Where the combined web **40** has the non-adhesive portion **12**, it is preferred that the embossing section **51** has at least two rows of protrusions such that a portion of a protrusion in the first row overlaps a portion of a protrusion in the second row in the direction of the rotation axis of the embossing roll **50**, in order to reduce or eliminate the shrinking force of the elastic member **20** on the design area **150**. This is because it is not possible, with a single row of protrusions, to cut off a portion of the elastic member **20** located between adjacent protrusions. Even with a single row of protrusions, the elastic member **20** can be cut off if the protrusions are in a slant arrangement. Alternatively, for example, cutting off, etc., of aplurality of rubber strings can be achieved with a single protrusion. The length L₂ of the embossing section **51** may be slightly smaller than the length L₁ of the non-adhesive portion **12**.

In underpants **140**, an adhesive is applied also in the design area **150**. In order to reduce or eliminate the shrinking force of the part of the elastic member **20** on the design area **150,** it is preferred that the protrusions in each row are arranged at a predetermined interval in the embossing section **51** across the entirety of the design area **150**. In other words, it is preferred that the protrusions have an overlap with one another as they are projected in the axial direction of the embossing roll **50**.

While the elastic member **20** exists under the sheet, the processing section **2** may be used to reduce or eliminate the shrinking force of the elastic member **20** in advance in cases where an additional member (e.g., a tape used when disposing of the underpants or a tape to be attached to a diaper body or a label) is adhered on the sheet. This is because it is difficult to fix such an additional member on the sheet while the elastic member **20** is shrunk.

Underpants **160** are made of a combined web having a meshed elastic member **170** sandwiched between the first and second sheets at a predetermined tension. In a waist area **161**, the vertical elastics are cut off while leaving the horizontal elastics (the elastic around the waist) so that the underpants **160** fit well to the body. Note however that the vertical and horizontal elastics are both cut off in an area **162** where an additional member or a label is adhered. Moreover, the vertical and horizontal elastics are both cut off in a predetermined area **163**. The predetermined area **163** is an area where it is preferred that the underpants **160** do not shrink. An absorber may be provided in the area.

As described above, it is made possible to adhere a member on the combined web **40** by reducing the shrinking force of the elastic member **20** of the combined web **40** in areas where the member is to be adhered. Possible methods for reducing or eliminating the shrinking force of the elastic member **20** include: cutting off the elastic member **20**; reducing the shrinking force of the elastic member **20**; altering the molecular structure of the elastic member **20** (when the elastic member **20** is a rubber), and melting a part of the combined web **40** and curing the melted part.

The protrusions may generate heat in order to reduce or eliminate the shrinking force of the elastic member. In such a case, the elastic member **20** is cut off by the protrusions melting at least a part of the second sheet **30** and at least a part of the elastic member **20**. In this process, the first sheet and the second sheet are sealed together. For example, in the case of the underpants **130**, the first sheet and the second sheet are sealed together while the elastic member **20** is cut off. The wrinkling of the elastic member **20** is improved when the shrinking force of the elastic member **20** is reduced, even if the elastic member **20** is not cut off.

Moreover, even if the protrusions do not generate heat, it is possible to cut off the elastic member **20** via the second sheet **30** when the width of each protrusion is small (e.g., several microns to about 0.5 mm). In this process, at least a part of the second sheet **30** is cut by the protrusions, thereby increasing the air permeability of the underpants **140**. In addition to the design area **150**, a part of the elastic member **20** corresponding to an upper waist area **151** and/or a leg area **152** of the underpants **140** may also be opened by the protrusions.

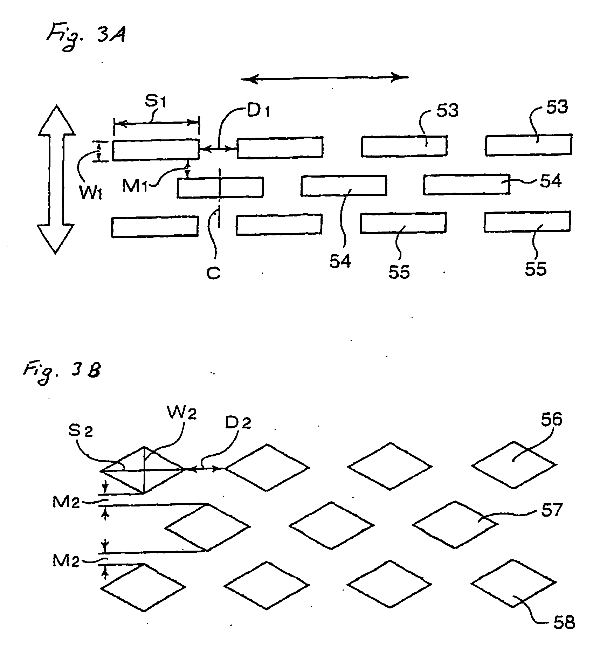

The protrusions of the embossing section **51** will now be described. FIG. **3A** illustrates an emboss pattern including a plurality of rectangular protrusions arranged in a staggered pattern. The embossing section **51** includes: an n-1^{th} row of rectangular protrusions **53, 53** each having a length S₁ and a width W₁ which are arranged in the axial direction of the embossing roll **50** (indicated by a solid arrow) at intervals of a distance D₁; an n^{th} row of protrusions **54, 54** each having the same length (S₁) and width (W₁) as those of the protrusions **53** which are arranged in the circumferential direction of the embossing roll **50** (indicated by an outline arrow) at a distance of M₁ from the protrusions 53 so that the longitudinal center line thereof passes through a point of D₁/2; and an n+1^{th} row of protrusions **55, 55** having the same shape as that of the protrusions **53** which are arranged in the same manner as the protrusions **53**. The number of protrusions in each row may be determined based on, for example, the number of elastic members to be cut off. The number of rows of protrusions may be determined based on the length L₁ of the non-adhesive portion. Herein, n is a natural number, and "0 row" means there is no row of protrusions.

In an alternative emboss pattern where n is a natural number equal to or greater than 2, a protrusion in the n-1^{th} row may overlap with the protrusion in the n+1^{th} row in the row direction by at least 1 mm or more. In such a case, a protrusion in the n-1^{th} row may not overlap with the protrusion in the n^{th} row or may overlap with the protrusion in the n^{th} row by about 0.5 mm to about 1 mm. In this way, the density of protrusions in each row may be reduced as compared to the emboss pattern illustrated in FIG. **3A**, thereby facilitating the production of the emboss roll.

With an emboss roll having such protrusions as described above, as compared to an emboss roll having a single line blade, the protrusions more easily cut into the combined web, whereby it is possible to easily cut off the elastic member.

FIG. **3B** shows a pattern in which diamond-shaped protrusions are arranged in a staggered pattern. The embossing section **51** includes: a row of diamond-shaped protrusions **56, 56** each having a long axis S₂ and a short axis W₂ which are arranged in the axial direction of the embossing roll **50** (indicated by a solid arrow) at intervals of a distance D₂; another row of protrusions **57, 57** each having the same diamond shape as the protrusions **56** which are arranged in the circumferential direction of the embossing roll **50** (indicated by an outline arrow) at a distance of M₂ from the protrusions **56** so that the short axis thereof is collinear with a point of D₂/2; and still another row of protrusions **58, 58** each having the same shape as that of the protrusions **56** which are arranged in the same manner as the protrusions **56.** Of course, also for this pattern, the number of protrusions in each rowmay be determined based on, for example, the number of elastic members to be cut off, and the number of rows of protrusions may be determined based on the length L₁ of the non-adhesive portion.

The length S₁ of the rectangular protrusions and the length of the long axis S₂ of the diamond-shaped protrusions are both preferably in the range of 1 mm to 25 mm, and more preferably 2 mm to 25 mm. Where the interval D₁ between adjacent protrusions is less than or equal to S₁, the elastic member located between adjacent protrusions **53** can be reliably cut off by the protrusion **54** due to the staggered arrangement. Similarly, D₂ and S₂ may be determined so that D₂≤S₂ holds. When S₁ or S₂ is less than 1 mm, the embossing section **51** may fail to cut off the elastic member, and when it is greater than 25 mm, the feel/touch of the article may deteriorate due to the excessive total area of seal portions. D₁ and D₂ are also in the range of 1 mm to 25 mm, and D₁ is more preferably 2 mm to 25 mm. Where diamond-shaped protrusions are used, if a protrusion in a row has little overlap with the closest protrusion in an adjacent row in the row direction, the elastic member may fall between adjacent seal portions, and the embossing section **51** may thereby fail to cut off the elastic member, depending upon the arrangement of the protrusions. In view of this, D₂ is more preferably 3 mm to 10 mm.

When a portion of the combined web is to be melted, the width W₁ of the rectangular protrusions and the length of the short axis W₂ of the diamond-shaped protrusions are preferably 0.5 mm to 15 mm. When the first sheet **10** and the elastic member are cut off, W₁ is preferably several microns to about 0.5 mm. When they are greater than 15 mm, the feel/touch of the manufactured article may deteriorate due to the excessive total area of seal portions. The lower limit of W₂ is preferably 1 mm or more.

While the distance between adjacent rows of protrusions is not limited to any particular value, M₁ or M₂ is preferably 1 mm to 25 mm. The protrusions may have a shape other than a rectangular shape and a diamond shape as described above, including a slanted rectangular shape, a circular shape, a triangular shape, a star shape, a heart shape, a clover shape, a crescent shape, other polygons, etc. The shape of the protrusions maybe varied for different rows.

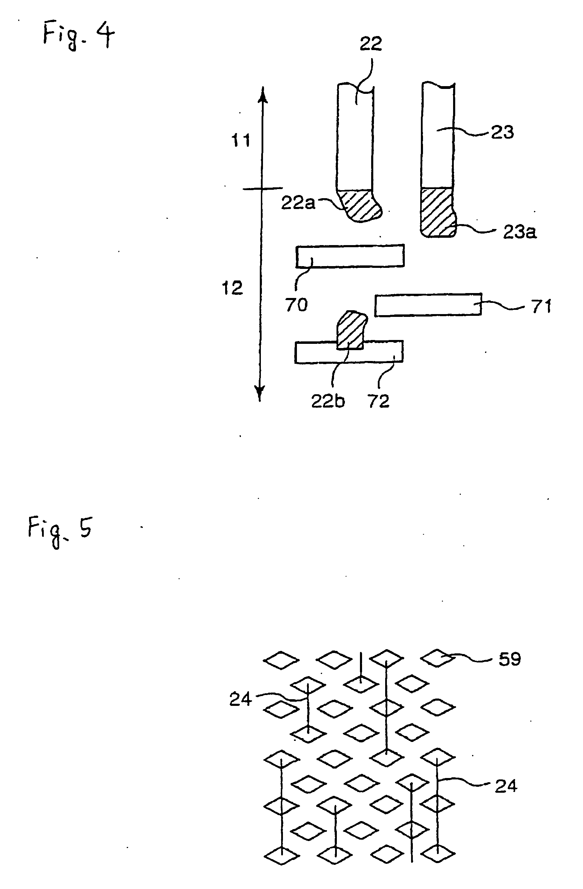

FIG. **4** schematically illustrates elastic members **22** and **23** having been cut off. It is assumed that the combined web is advanced downwardly in FIG. **4**. The area **11** is the adhesive portion and the area **12** is the non-adhesive portion. The left-hand side elastic member **22** is cut off by a seal portion **70** (corresponding to the protrusion **53**). An end portion **22a** of the elastic member **22** is released from the tension and shrink toward the elastic member **22** bonded on the sheet. If the remaining part of the elastic member is caught by the protrusion **55** before completion of the cutting off by the protrusion **53**, an elastic member **22b** between the protrusion **53** and the protrusion **55** shrinks toward a seal portion **72** when the elastic member is cut off by the protrusion **53**. If the cutting off by the protrusion **53** is completed before the remaining part of the elastic member is caught by the protrusion **55**, the elastic member **22b** will shrink toward the elastic member existing in the downstream adhesive portion (not shown). The right-hand side elastic member **23** is cut off by a seal portion **71** (corresponding to the protrusion **54**), and an end portion **23a** thereof shrinks.

With the arrangement described above, the elastic member is cut off and a large number of small seal portions are formed in the non-adhesive portion, whereby the upper and lower sheets are bonded together also in the non-adhesive portion. Since the seal portions are separated from one another and each have a small size, they are less likely to give discomfort to the wearer than when they are provided as a continuous line even when they are heat-sealed into a film.

An exemplary adhesive applicator will now be described.

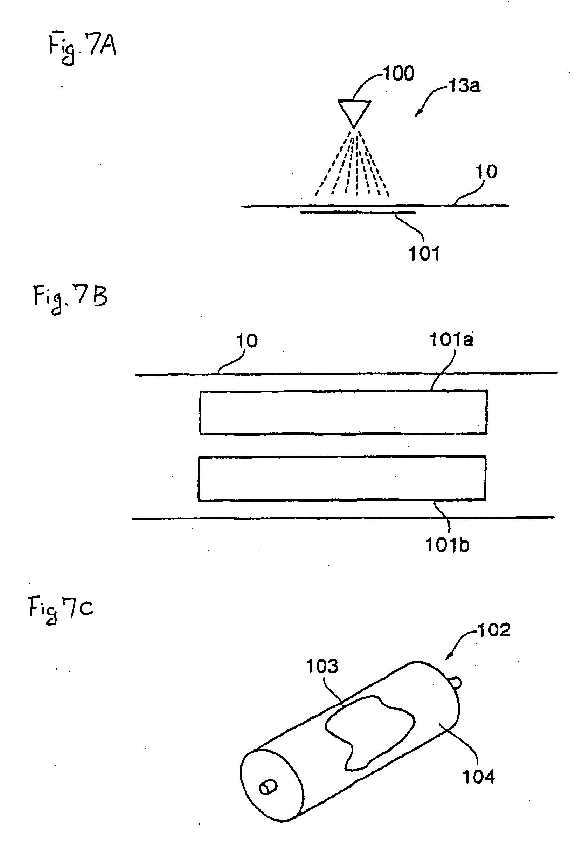

FIG. **7A** is a diagram illustrating an exemplary adhesive applicator **13a.** The adhesive applicator **13a** includes a gun member **100** for spraying an adhesive (a hot melt in the following description) while positively charging the hot melt, and a conductive member **101** which is located under the first sheet **10** and is grounded or negatively charged. Generally, not all of the hot melt sprayed from the gun member **100** is attached to the sheet, but a portion thereof is left floating in the air. However, by charging the hot melt as described above, it is possible to efficiently attach the hot melt to the sheet, and to reduce the amount of hot melt to be scattered with respect to the width direction of the sheet, thereby improving the widthwise precision. Avoltage of the same polarity as the hot melt may be applied to portions where it is not necessary to apply the hot melt.

It is possible to improve the positional precision of the attachment of the hot melt in the flow direction, which is transverse to the width direction, by controlling the period in which the hot melt is output and the potential of the gun member **100** or the conductive member **101.** The polarities of the applied voltages may be reversed from that described above.

The conductive member **101** may be provided in the form of a plurality of plates **101a** and **101b** as illustrated in FIG. **7B**. For example, when the hot melt is applied on areas separated by a predetermined interval by using a plurality of gun members **100**, the hot melt application may be performed with the interval between adjacent hot melt-applied areas being the interval between the plates **101a** and **101b**.

Alternatively, the conductive member **101** may be provided in the form of a roller **102** as illustrated in FIG. **7C****.** The roller **102** rotates in synchronism with the first sheet **10**. The roller **102** includes a conductive portion **103** and a non-conductive portion **104**. As described above, the hot melt is charged to a potential of the first polarity, and the conductive portion **103** is grounded or charged to a potential of the opposite polarity to the first polarity.

The shape of the conductive portion **103** is determined based on the shape of the area of the first sheet **10** on which the hot melt is to be applied. The gun member **100** is controlled to output the hot melt a predetermined time before the leading edge of the area on which the hot melt is to be applied passes by the gun member **100,** and to stop outputting the hot melt a predetermined time before the trailing edge of the area on which the hot melt is to be applied passes by the gun member **100.** With such an arrangement, it is possible to attach the hot melt to an area substantially equal to the intended area on which the hot melt should be applied. The roller **102** may be positioned upstream of the gun member **100.**

It is understood that the adhesive applicator as described above may be used for manufacturing articles other than disposable worn articles. Nothing may be sandwiched between the first sheet and second sheet after the application of the hot melt, or a member other than the web materials may be adhered by the hot melt after the application of the hot melt.

The hot melt adhesive may be continuously applied without providing a non-adhesive portion. This may be a more preferred embodiment than when the adhesive portions and the non-adhesive portions are alternately provided, because it is possible, in the former embodiment, to further increase the production line speed. The elastic member may be sandwiched between sheets on which an adhesive has been continuously applied and then passed between the embossing roll and the counter roll, so as to cut off only the elastic member without cutting off the sheets. After the cutting process, each elastic member **24** is held by two (or three or more) seal portions **59** and **59**, as illustrated in FIG. **5****,** while being relaxed. Therefore, even when the coating of the hot melt adhesive is continuous, the seal portion can be made non-elastic by changing the type and/or amount of the hot melt adhesive and/or the spraying direction thereof. It is believed that where the elastic member is cut off by heat, the hot melt adhesive is re-melted and softened by the heat of the embossing roll, so that the anchoring force on the elastic member by the hot melt adhesive is reduced and the elastic recovering force of the elastic member overcomes the anchoring force, whereby the elastic member is bonded to the seal portion while being relaxed.

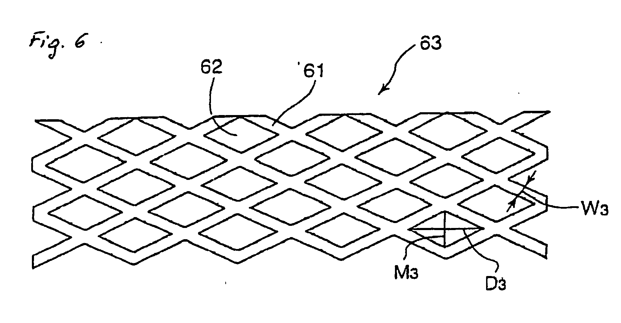

Next, a second embodiment of the present invention will be described. The second embodiment employs, instead of the embossing roll having protrusions spaced apart from one another, an embossing roll including depressions having a length of 1 mm to 25 mm and a width of 0.5 mm to 15 mm and a latticed protrusion (ridges) having a width of 0.5 mm to 5 mm. As illustrated in FIG. **6**, the latticed protrusion **63** includes a large number of ridges **61, 61**, ..., which are arranged in the form of parallel crosses, with each space **62** surrounded by the ridges **61** being a depression. W₃ denotes the width of each ridge, which is 0.5 mm to 5 mm. An excessively large width of the ridges may deteriorate the feel/touch of the manufactured article, while the ridges having a width smaller than 0.5 mm may possibly cut off the sheet. D₃ is the length of each depression, which is preferably 5 mm to 25 mm, and more preferably 5 mm to 10 mm. M₃ is the width of each depression, which is preferably 5 mm to 25 mm, and more preferably 5 mm to 10 mm.

While FIG. **6** shows a slanted lattice pattern, the pattern may alternatively be any other polygonal lattice pattern such as a square lattice pattern or a rectangular lattice pattern. In this embodiment, the seal portions are in a lattice pattern, whereby it is possible to reliably cut off the elastic member. Moreover, since the latticed protrusion made up of narrow ridges extends across a sufficiently large area in a meshed pattern, the elastic member can be cut off by any of the seal portions. Where the sealing is provided by using a single line blade, all the elastic members need to be cut off by the single line blade, whereby it is necessary to perform strong sealing so that no elastic member is left uncut. As a result, the sheet may possibly be cut off. With the above-described arrangement, the elastic member can be cut off by any of the seal portions. Therefore, the sheet will not be cut off because it is not necessary to perform strong sealing and the contact surface between the sheet and the protrusion is increased. Moreover, such a latticed seal portion gives the wearer a soft feel/touch, and also is aesthetically desirable. Also in the second embodiment, the sheet may include hot melt adhesive portions and non-adhesive portions, or the hot melt adhesive may be continuously applied on the sheet without providing the non-adhesive portions.

At least one of the embossing roll **50** and the counter roll **60** illustrated in FIG. **1** may include a heating member. Moreover, other heating members such as a rod-shaped sheathed heater, a high-frequency heating member, a far infrared heater, or an oil heater, may be additionally provided in the vicinity of these rolls. In the arrangement of FIG. **1****,** the positions of the embossing roll **50** and the counter roll **60** may be reversed.

It is preferred that either the first sheet **10** or the second sheet **30** is heat sealable. Applicable types of sheet include non-woven fabric, a plastic film, knit fabric, woven fabric, paper, etc. Applicable sheet materials include known materials such as a polypropylene, a polyethylene, a polyester, a cellulose, a rayon, etc., which can be used alone or in combination of two or more. Each of the sheets **10** and **30** may be a multi-layer sheet including a number of sheets laminated together. In such a case, a heat sealable sheet should be provided on the uppermost surface of the first sheet **10** or on the lowermost surface of the second sheet **30.**

The elastic member may be made of a material that can be cut off by heat (e.g., a thermoplastic polyurethane, an elastomer, a rubber, etc.), and may be provided in the form of a ribbon or a string. A film-shaped elastic member (e.g., an elastomer film) can also be used because it can be reliably cut off by seal portions arranged in a staggered pattern or a lattice pattern. It is preferred that the elastic member has a melting point that is lower than that of the heat sealable material of the second sheet so that the second sheet will not be cut off. The melting point of the second sheet may be higher than that of the first sheet.

While FIG. **1** illustrates an example where the elastic member is adhered in the widthwise central portion of the sheet, the elastic member may alternatively be adhered near the edge of the sheet. In such a case, the emboss pattern can be changed according to the position of the elastic member .

According to the present invention, the seal portions can be made non-elastic after the heat seal process not only in the case where an adhesive is applied so as to provide non-adhesive portions and adhesive portions but also in the case where the adhesive application is continuous. Therefore, an elastic sheet having alternating elastic and non-elastic portions can be continuously manufactured.

## Claims

1. A method for manufacturing a combined web (40) for a disposable worn article, the method comprising:
a first step of applying an adhesive on at least one of a first web (10) and a second web (30);
a second step of sandwiching an elastic member (20) between the first and second webs (10, 30) and combining the first and second webs (10, 30) and an elastic member (20) together, thereby producing a combined web (40) wherein the adhesive is placed between the first web (10) and the second web (30); **characterized by**
a third step of cutting off a portion of at least one of the first and second webs (10, 30), and the elastic member (20) by passing the combined web (40) between an embossing roll (50) having a plurality of protrusions (53, 54, 55, 63) which are arranged in the axial direction of the embossing roll at intervals, and a counter roll (60),
wherein:
an interval of the protrusions (53-55) in a direction of a rotation axis of the embossing roll (50) is about 1 mm to 25 mm.

2. A method for manufacturing a disposable worn article according to claim 1, wherein:
at least the first web (10) includes a design area (150) having at least one of a graphical design, a symbol and a character printed thereon;
and
the elastic member (20) located on at least a portion of the design area (150) is cut off in the third step.

3. A method for manufacturing a disposable worn article according to any one of claims 1 to 2, wherein:
at least the second web (30) includes an area (162) on which a member is to be adhered; and
the elastic member (20) located under at least a portion of the area (162) is cut off in the third step.

4. A method for manufacturing a disposable worn article according to any one of claims 1 to 3, wherein the elastic member (20) is at least one of a string rubber, a flat rubber and a meshed rubber.

## Patentansprüche

1. Ein Verfahren zur Herstellung des kombinierten Gewebes (40) für einen wegwerfbaren getragenen Artikel, das Verfahren aufweisend:
einen ersten Schritt des Aufbringens eines Klebstoffs auf zumindest eines aus einem ersten Gewebe (10) und einem zweiten Gewebe (30);
einen zweiten Schritt des Einlegens eines elastischen Elements (20) zwischen das erste und zweite Gewebe (10, 30) und des Zusammenkombinierens des ersten und zweiten Gewebes (10, 30) und eines elastischen Elements (20), dabei herstellend ein kombiniertes Gewebe (40), wobei der Klebstoff zwischen dem ersten Gewebe (10) und dem zweiten Gewebe (30) platziert ist; **gekennzeichnet durch**
einen dritten Schritt des Abschneidens eines Teils von zumindest einem aus dem ersten und dem zweiten Gewebe (10, 30), und dem elastischem Element (20) **durch** Hindurchführen des kombinierten Gewebes (40) zwischen einer Prägerolle (50) mit einer Vielzahl von Vorsprüngen (53, 54, 55, 63), die in der axialen Richtung der Prägerolle in Intervallen angeordnet sind, und einer Gegenrolle (60), wobei:
ein Intervall der Vorsprünge (53 - 55) in einer Richtung einer Drehachse der Prägerolle (50) circa 1 mm bis 25 mm ist.

2. Ein Verfahren zur Herstellung eines wegwerfbaren getragenen Artikels nach Anspruch 1, wobei
zumindest das erste Gewebe (10) einen Designbereich (150) enthält, auf dem zumindest eins aus einem graphischen Design, einem Symbol und einem Buchstaben aufgedruckt ist; und
das elastische Element (20) angeordnet auf zumindest einem Teil des Designbereichs (150) in dem dritten Schritt abgeschnitten wird.

3. Ein Verfahren zur Herstellung eines wegwerfbaren getragenen Artikels nach einem der Ansprüche 1 bis 2, wobei:
zumindest das zweite Gewebe (30) einen Bereich enthält, auf dem ein Element anzukleben ist; und
das elastische Element (20) gelegen unter zumindest einem Teil des Bereichs (162) in dem dritten Schritt abgeschnitten wird.

4. Ein Verfahren zur Herstellung eines wegwerfbaren getragenen Artikels nach einem der Ansprüche 1 bis 3, wobei das elastische Element (20) zumindest eines ist aus einer Gummischnur, einem flachen Gummi und einem netzartigen Gummi.

## Revendications

1. Procédé de fabrication d'une bande de tissu combinée (40) pour un article à porter jetable, le procédé comprenant:
une première étape d'application d'un adhésif sur au moins une d'une première bande de tissu (10) et d'une deuxième bande de tissu (30);
une deuxième étape consistant à coincer un élément élastique (20) entre les première et deuxième bandes de tissu (10, 30), et à combiner les première et deuxième bandes de tissu (10, 30) et un élément élastique (20) ensemble, produisant de ce fait une bande de tissu combinée (40), dans lequel l'adhésif est placé entre la première bande de tissu (10) et la deuxième bande de tissu (30);
**caractérisé par**:
une troisième étape de coupe d'une partie d'au moins une des première et deuxième bandes de tissu (10, 30) et de l'élément élastique (20) en faisant passer la bande combinée (40) entre un rouleau de gaufrage (50) comportant une pluralité de saillies (53, 54, 55, 63) qui sont agencées dans la direction axiale du rouleau de gaufrage à des intervalles, et un contre-rouleau (60), dans lequel un intervalle entre les saillies (53-55) dans une direction d'un axe de rotation du rouleau de gaufrage (50) est d'environ 1 mm à 25 mm.

2. Procédé de fabrication d'un article à porter jetable selon la revendication 1, dans lequel:
au moins la première bande de tissu (10) comprend une zone de dessin (150) comportant au moins une représentation graphique, un symbole et un caractère imprimés sur celle-ci; et
l'élément élastique (20) situé sur au moins une partie de la zone de dessin (150) est coupé lors de la troisième étape.

3. Procédé de fabrication d'un article à porter jetable selon l'une quelconque des revendications 1 à 2, dans lequel:
au moins la deuxième bande de tissu (30) comprend une zone (162) sur laquelle un élément doit être collé; et
l'élément élastique (20) situé en dessous d'au moins une partie de la zone (162) est coupé lors de la troisième étape.

4. Procédé de fabrication d'un article à porter jetable selon l'une quelconque des revendications 1 à 3, dans lequel l'élément élastique (20) est au moins un élément parmi un caoutchouc ficelle, un caoutchouc plat et un caoutchouc maillé.
